# EUROPÄISCHE PATENTANMELDUNG

(11) **EP 2 057 979 A1**
(43) Veröffentlichungstag der Anmeldung: **13.05.2009**
(21) Anmeldenummer: 08019417.8
(22) Anmeldetag: 06.11.2008
(51) Int. Cl.: A61K 8/60, A61Q 5/04, A61Q 5/08, A61Q 5/10

(54) **Farbige Haarbehandlungsmittel**

(30) Priorität: 18.08.2008 DE 102008038138; 09.11.2007 DE 102007053950; 09.11.2007 DE 102007053952
(71) Anmelder: Henkel AG & Co. KGaA, 40589 Düsseldorf (DE)
(72) Erfinder: Rath, Susanne, 20359 Hamburg (DE); Kleen, Astrid, 20457 Hamburg (DE)

(57) **Zusammenfassung**

Die vorliegende Erfindung betrifft Mittel zum Färben keratinischer Fasern, die mindestens ein Farbstoffvorprodukt sowie ein Bräunungsprodukt, das durch Erhitzung aus Zucker gewonnen wird, enthalten und einen pH-Wert von 7 bis 14, vorzugsweise von 9 bis 12 aufweisen.

## Beschreibung

Die vorliegende Anmeldung betrifft Mittel zur Färbung keratinische Fasern, die ein aus Zucker gewonnenes Bräunungsprodukt enthalten, ein Färbeverfahren unter Einsatz dieser Mittel sowie die Verwendung der Bräunungprodukte als Anfärbefarbstoff sowie zur Maskierung unangenehmer Gerüche in haarkosmetischen Zubereitungen.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und

Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Konventionelle Haarfärbemittel enthalten als farbverändernde Wirkstoffe in der Regel mindestens eine Entwickler- und/oder mindestens eine Kupplerkomponenten und gegebenenfalls noch direktziehende Farbstoffe als Nuanceure. Kuppler- und Entwicklerkomponenten werden auch als Oxidationsfarbstoffvorprodukte bezeichnet. Vor ihrer Anwendung auf menschliches Haar werden üblicherweise Haarfärbe- und/oder -aufhellungsmittel in fester oder pastöser Form mit einer verdünnten wässrigen Wasserstoffperoxid-Lösung vermischt. Die resultierende Mischung wird dann auf das Haar aufgebracht und nach einer bestimmten Einwirkzeit wieder ausgespült. Die Einwirkungsdauer auf dem Haar zur Erzielung einer vollständigen Ausfärbung beziehungsweise Aufhellung liegt in der Regel zwischen etwa 30 und 40 Minuten. Es ist nahe liegend, dass bei den Benutzern dieser Haarfarben oder Blondiermittel ein Bedürfnis besteht, diese Einwirkungszeit zu verringern.

Um eine ausreichenden Färbewirkung zu erzielen, sind derartige Mittel üblicherweise stark alkalisch eingestellt, der pH-Wert liegt dabei zwischen 8 und 10,5. Derart hohe pH-Werte sind erforderlich, um eine Öffnung der äußeren Schuppenschicht (Cuticula) zu gewährleisten und somit eine Penetration der aktiven Spezies (Wasserstoffperoxid) ins Haar zu ermöglichen.

Das äußere Erscheinungsbild der färbenden Mittel wird maßgebliche durch die Konfektionierungsform, zum Beispiel als Emulsion oder Gel, sowie durch die enthaltenen Farbstoffe geprägt. Als solche leisten zum einen die in den Formulierungen enthaltenen direktziehenden Farbstoffe einen Beitrag. Um aber einer gesamten Produktlinie auch unabhängig von der exakten Formulierung der einzelnen Nuancen eine einheitliche Färbung zu verleihen, werden häufig chemische Anfärbefarbstoffe eingesetzt. Da derartige Farbstoffe hinsichtlich ihres Allergiepatentioals sowie ihrer biologischen Abbaubarkeit noch Wünsche offenlassen, bestand der Bedarf an milden, natürlichen Anfärbefarbstoffen für haarkosmetische Zubereitungen, insbesondere für oxidative Färbemittel.

Ein weiterer Aspekt der vorliegenden Erfindung ist das Bestreben, das Geruchserlebnis bei der haarkosmetischen Behandlung zu verbessern. Viele der üblicherweise verwendeten haarkosmetischen Mittel enthalten Bestandteile, die einen häufig als unangenehm empfundenen Duft aufweisen. Es bestand daher die Aufgaben einen Zusatzstoff zu entwickeln, der in der Lage ist, die als unangenehm empfundenen Gerüche zu maskieren.

Die im Rahmen dieser Aufgabestellungen einsetzbaren Wirkstoffe müssen insbesondere hinsichtlich ihrer Stabilität hohen Ansprüchen genügen, da die haarkosmetischen Zubereitungen extreme pH-Bereiche aufweisen. So sind beispielsweise die Färbecremes und Wellmittel durch einen hohen pH-Wert und die Oxidationsmittelzubereitungen durch einen niedrigen pH-Wert gekennzeichnet. Auch müssen diese Wirkstoffe gegenüber den reduzierenden Bedingungen der Wellmittel stabild sein. Ferner sind Unverträglichkeiten der verschiedenen Wirkstoffe untereinander und somit eine geringe Lagerstabilität zu vermeiden.

Es bestand daher weiterhin Bedarf an Wirkstoffen, die als Anfärbefarbstoff stabil in haarkosmetischen Zubereitungen eingesetzt werden können und sich darüber hinaus durch eine gute biologische Abbaubarkeit, geringe Rohstoffkosten sowie eine gute Verträglichkeit auszeichnen. Weiterhin sollte dieser Wirkstoff auch einen positiven Einfluß auf den Pflegezustand der Haare ausüben.

Es wurde nunmehr überraschenderweise gefunden, dass Mittel zur Färbung keratinischer Fasern mittels spezieller Bräunungsprodukte, die aus Zucker gewonnen werden, stabil angefärbt werden können. Gleichzeitig wurde gefunden, dass der als unangenehm empfundene Geruch derartiger Mittel durch den Einsatz dieser Bräunungsprodukte maskiert werden kann.

Ein erster Gegenstand der vorliegenden Anmeldung ist daher ein Mittel zur Färbung keratinischer Fasern, das mindestens ein Farbstoffvorprodukt sowie ein Bräunungsprodukt, das durch Erhitzung aus Zucker gewonnen wird, enthält und einen pH-Wert von 7 bis 14, vorzugsweise von 9 bis 12 aufweist.

Unter "keratinischen Fasern" sind dabei erfindungsgemäß Pelze, Wolle, Federn und insbesondere menschliche Haare zu verstehen.

Als ersten erfindungswesentlichen Bestandteil enthalten die Färbemittel mindestens ein Farbstoffvorprodukt.

Hinsichtlich der in den erfindungsgemäßen Färbemitteln einsetzbaren Farbstoffvorprodukte unterliegt die vorliegende Erfindung keinerlei Einschränkungen. Die erfindungsgemäßen Färbemittel können als Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

Im Rahmen einer ersten bevorzugten Ausführungsform der vorliegenden Erfindung enthalten die erfindungsgemäßen Mittel mindestens ein Farbstoffvorprodukt vom Entwickler- und/oder Kupplertyp.

Es kann erfindungsgemäß bevorzugt sein, als Entwicklerkomponente ein p-Phenylendiaminderivat oder eines seiner physiologisch verträglichen Salze einzusetzen.

Besonders bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-Methoxymethyl-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, 2-Methoxymethyl-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Bevorzugte zweikernige Entwicklerkomponenten werden insbesondere aus mindestens einer der folgenden Verbindungen ausgewählt: N,N'-Bis-(β-hydroxyethyl)-N,N'-bis-(4'-aminophenyl)-1,3-diaminopropan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen.

Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, sowie deren physiologisch verträglichen Salze.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Aminobenzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-

Ethylamino-4-methylphenol, 2,4-Dichlor-3-aminophenol und den physiologisch verträglichen Salzen aller vorstehend genannten Verbindungen.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und den physiologisch verträglichen Salzen aller vorstehend genannten Verbindungen.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-

Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Weiterhin können die Mittel zusätzlich zu den Farbstoffvorprodukten mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

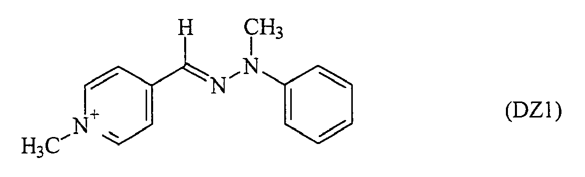

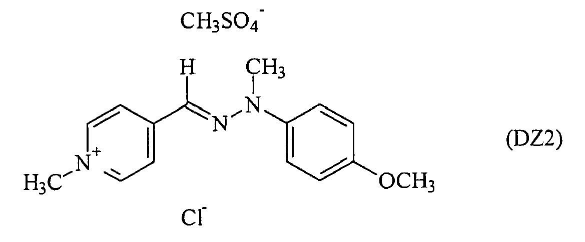

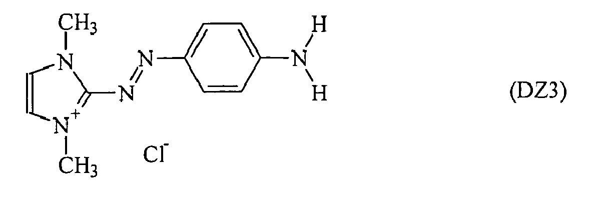

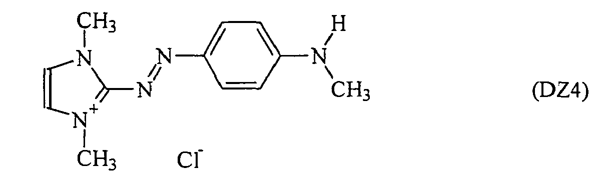

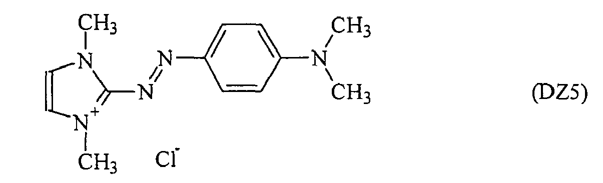

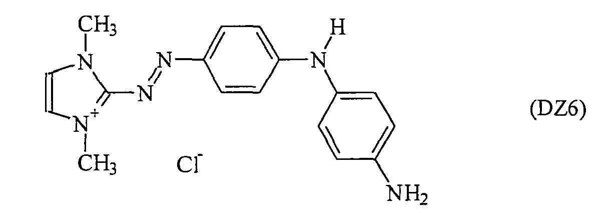

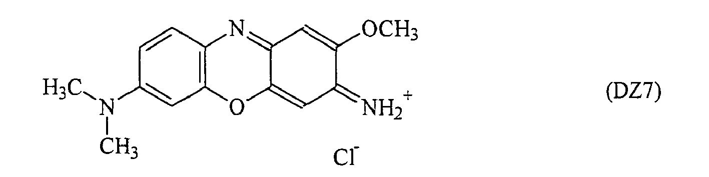

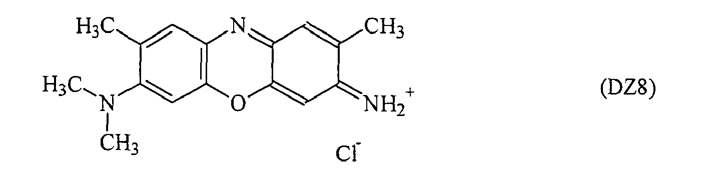

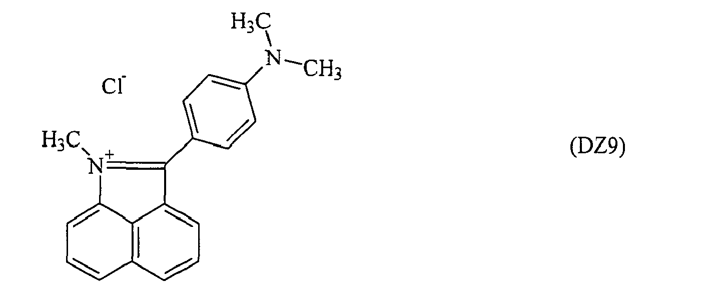

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe. Nichtionische direktziehende Farbstoffe:

Als nichtionische direktziehende Farbstoffe eignen sich insbesondere nichtionische Nitro- und Chinonfarbstoffe und neutrale Azofarbstoffe.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Die erfindungsgemäßen Mittel enthalten die direktziehenden Farbstoffe bevorzugt in einer Menge von 0,01 bis 20 Gew.-%, bezogen auf die gesamte Anwendungszubereitung.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Als zweiten wesentlichen Bestandteil enthalten die erfindungsgemäßen Mittel mindestens ein Bräunungsprodukt, das durch Erhitzung aus Zucker gewonnen wird.

Unter dem Begriff "Zucker" werden erfindungsgemäß organische Verbindungen verstanden, die eine Carbonylgruppe sowie mehrere Hydroxygruppen tragen. Dabei sind erfindungsgemäß auch solche Verbindungen umfasst, bei denen die Carbonylgruppe eine Halbacetal- oder Halbketalgruppe bildet. Erfindungsgemäß bevorzugt sind sowohl Monosaccharide, Disaccharide, Trisaccharide als auch Polysaccharide. Besonders bevorzugt Zuckerarten sind Saccharose, invertierte Saccharose, Dextrose (D-Glucose) und Fructose (D-Fructose). Saccharose ist ein erfindungsgemäß ganz besonders bevorzugter Zucker.

Das erfindungsgemäße Bräunungsprodukt wird aus dem Zucker durch Erhitzen gewonnen.

In einer ersten bevorzugten Ausführungsform wird das Bräunungsprodukt aus Polysacchariden durch Zusatz eines oder mehrerer Bräunungsbeschleunigern gewonnen. Bräunungsbeschleuniger sind erfinudngsgemäß Substanzen, die dem Zucker vor dem Erhitzen zugegeben werden, um die Bräunung gezielt zu beeinflussen und gegebenenfalls zu beschleunigen.

Als solche werden erfindungsgemäß bevorzugt Carbonate, Hydroxide, Säuren, Sulfite, Ammoniumverbindungen sowie Schwefeldioxid verwendet.

Erfindungsgemäß besonders bevorzugte Bräunungsbeschleuniger sind Natriumcarbonat, Kaliumcarbonat, Natriumhydroxid, Kaliumhydroxid, Essigsäure, Citronensäure, Schwefelsäure, Schwefeldioxid, Ammoniak, Ammoniumcarbonat, Ammoniumsulfit, Natriumsulfit und/oder Kaliumsulfit.

Unter Zuhilfenahme dieser Bräunungsbeschleuniger werden aus dem Zucker Brünungsprodukte erhalten die in der Lebensmittelchemie unter den Bezeichnungen E 150a, E 150b, E 150c und/oder E 150d bekannt sind.

In einer zweiten Ausführungsform der vorliegenden Erfindung wird das Bräunungsprodukt ausschließlich durch Erhitzen eines oder mehrerer Zuckerarten ohne Zugabe eines Bräunungsbeschleunigers gewonnen wird. Ein Beispiel für ein besonders bevorzugtes Bräunungsprodukt, das ohne Zusatz von Bräunungsbeschleunigern erhalten wird, ist Karamel.

Als drittes erfindungswesentliches Merkmal weisen die Mittel zur Färbung keratinischer Fasern einen alkalischen pH-Wert auf. Ein pH-Bereich von 7-14, insbesondere von 9 bis 12 ist erfindungsgemäß bevorzugt.

Zu diesem Zweck können die erfindunsgemäßen Mittel mindestens ein Alkalisierungsmittel enthalten. Obwohl prinzipiell jedes in der Kosmetik üblicherweise eingesetzte Alkalsierungsmittel erfindungsgemäß geeignet ist, haben sich die Mittel als besonder bevorzugt erwiesen, die ein Alkalisierungsmittel ausgewählt aus Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol, Triethanolamin, Arginin, Alkali- und Erdalkalimetallhydroxiden enthalten. Monoethanolamin und Arginin sind erfindungsgemäß ganz besonders bevorzugte Alkalisierungsmittel.

Es hat sich erfindungsgemäß als vorteilhaft erwiesen, wenn die Mittel der vorliegenden Erfindung einen Wirkstoff enthalten, der pflegende, entzündungshemmende, anti-irritierende und/oder hautberuhigende Eigenschaften aufweist.

Im Rahmen einer ersten bevorzugten Ausführungsform enthält das erfindungsgemäße farbverändernde Mittel als Pflegestoff mindestens ein kationisches Tensid.

Erfindungsgemäß bevorzugt sind kationische Tenside vom Typ der quarternären Ammoniumverbindungen, der Esterquats und der Amidoamine.

Die kationischen Tenside sind in den erfindungsgemäß verwendeten Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Im Rahmen einer zweiten bevorzugten Ausführungsform der vorliegenden Erfindung enthalten die farbverändernden Mittel als Pflegestoff mindestens ein pflegendes Polymer.

Die erfindungsgemäßen farbverändernden Mittel enthalten die kationischen Polymere bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Im Rahmen einer dritten bevorzugten Ausführungsform enthalten die erfindungsgemäßen farbverändernden Mittel mindestens einen UV-Filter. Die erfindungsgemäß geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyldimethylaminobenzamidopropyl-dimethylammoniumtosylat (Escalol^{®} HP 610).

Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt. Die UV-Filter sind in den erfindungsgemäßen Mitteln üblicherweise in Mengen 0,01-5 Gew.-%, bezogen auf die gesamte Anwendungszubereitung, enthalten. Mengen von 0,1-2,5 Gew.-% sind bevorzugt. Im Rahmen einer vierten bevorzugten Ausführungsform enthalten die erfindungsgemäßen farbverändernden Mittel als Pflegestoff mindestens ein Vitamin, ein Provitamin, eine Vitaminvorstufe sowie eines derer Derivate.

Dabei sind erfindungsgemäß solche Vitamine, Pro-Vitamine und Vitaminvorstufen bevorzugt, die üblicherweise den Gruppen A, B, C, E, F und H zugeordnet werden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-%, jeweils bezogen auf die gesamte Anwendungszubereitung enthalten.

Bevorzugt enthalten die erfindungsgemäßen farbverändernden Mittel Vitamine, Provitamine und Vitaminvorstufen aus den Gruppen A, B, C, E und H. Das Vitamin C kann erfindungsgemäß ganz besonders bevorzugt sein.

Panthenol, Pantolacton, Pyridoxin und seine Derivate sowie Nicotinsäureamid und Biotin sind besonders bevorzugt.

Im Rahmen einer fünften bevorzugten Ausführungsform enthalten die erfindungsgemäßen farbverändernden Mittel mindestens einen Pflanzenextrakt.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Besonders bevorzugt sind die Extrakte aus Moringa Olifeira, Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders geeignet sind die Extrakte aus Moringa Olifeira, Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und

Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen farbverändernden Mitteln Mischungen aus mehreren, insbesondere aus zwei, verschiedenen Pflanzenextrakten einzusetzen.

Im Rahmen einer sechsten Ausführungsform enthalten die erfindungsgemäßen farbverändernden Mittel als Pflegestoff mindestens eine Carbonsäure.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen.

Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-

Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 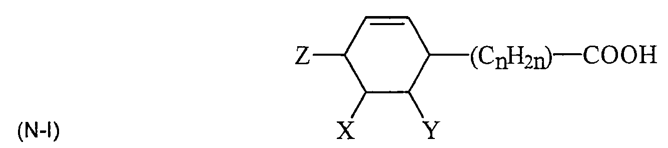 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt 2-Pyrrolidinon-5-carbonsäure und deren Derivate als Carbonsäure einzusetzen. Besonders bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf die gesamte Anwendungszubereitung, besonders bevorzugt 0,1 bis 5, und insbesondere 0,1 bis 3 Gew.%.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, dass neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure.

Im Rahmen einer siebten bevorzugten Ausführungsform enthalten die erfindungsgemäßen farbverändernden Mittel als Pflegestoff mindestens ein Proteinhydrolysat und eines seiner Derivate. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Die Proteinhydrolysate sind in den erfindungsgemäßen farbverändernden Mitteln in Konzentrationen von 0,01 Gew. -% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Im Rahmen einer achten bevorzugten Ausführungsform enthalten die erfindungsgemäßen Zubereitungen als Pflegestoff Ectoin oder Ectoinderivate, Allantoin, Taurin und Bisabolol enthalten.

Erfindungsgemäß werden unter dem Begriff "Ectoin und Ectoinderivate" Verbindungen der Formel (IV) 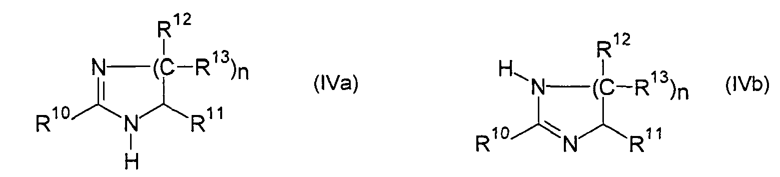 und/oder deren physiologisch verträglichen Salzes und/oder einer isomeren oder stereoisomeren Form verstanden, wobei
- R¹⁰ steht für ein Wasserstoffatom, einen verzweigten oder unverzweigten C₁ - C₄-Alkylrest oder einen C₂ - C₄-Hydroxyalkylrest,
- R¹¹ steht für ein Wasserstoffatom, eine Gruppierung -COOR¹⁴ oder eine Gruppierung - CO(NH)R¹⁴, wobei R¹⁴ für ein Wasserstoffatom, einen C₁ - C₄-Alkylrest, einen Aminosäurerest, einen Dipeptid- oder einen Tripeptidrest stehen kann,
- R¹² und R¹³ stehen unabhängig voneinander für ein Wasserstoffatom, einen C₁ - C₄-Alkylrest oder einer der beiden Reste steht für eine Hydroxygruppe und
- n steht für eine ganze Zahl von 1 bis 3.

Geeignete physiologisch verträgliche Salze der allgemeinen Verbindungen gemäß der Formel (IVa) oder (IVb) sind beispielsweise die Alkali-, Erdalkali-, Ammonium-, Triethylamin- oder Tris-(2-hydroxyethyl)aminsalze sowie solche, die sich aus der Umsetzung von Verbindungen gemäß der Formel (IVa) oder (IVb) mit anorganischen und organischen Säuren wie Salzsäure, Phosphorsäure, Schwefelsäure, verzweigten oder unverzweigten, substituierten oder unsubstituierten (beispielsweise durch eine oder mehrere Hydroxygruppen) C₁ - C₄- Mono- oder Dicarbonsäuren, aromatische Carbonsäuren und Sulfonsäuren wie Essigsäure, Citronensäure, Benzoesäure, Maleinsäure, Fumarsäure, Weinsäure und p-Toluolsulfonsäure ergeben. Beispiele für besonders bevorzugte physiologisch verträgliche Salze sind die Na-, K-, Mg- und Ca- und Ammoniumsalze der Verbindungen gemäß der Formel (IVa) oder (IVb), sowie die Salze, die sich durch Umsetzung von Verbindungen gemäß der Formel (IVa) oder (IVb) mit Salzsäure, Essigsäure, Citronensäure und Benzoesäure ergeben.

Unter isomeren oder stereoisomeren Formen der Verbindungen gemäß Formel (IVa) oder (IVb) werden erfindungsgemäß alle auftretenden optischen Isomere, Diastereomere, Racemate, Zwitterionen, Kationen oder Gemische davon verstanden.

Unter dem Begriff Aminosäure werden die stereoisomeren Formen, z.B. D- und L-Formen, folgender Verbindungen verstanden:

Asparagin, Arginin, Asparaginsäure, Glutamin, Glutaminsäure, β-Alanin, γ-Aminobutyrat, N_{ε}-Acetyllysin, N_{δ}-Acetylornitin, N_{γ}-Acetyldiaminobutyrat, N_{α}-Acetyldiaminobutyrat, Histidin, Isoleucin, Leucin, Methionin, Phenylalanin, Serin, Threonin und Tyrosin.

L-Aminosäuren sind bevorzugt. Aminosäurereste leiten sich von den entsprechenden Aminosäuren ab. Die folgenden Aminosäurereste sind bevorzugt:

Gly, Ala, Ser, Thr, Val, β-Ala, γ-Aminobutyrat, Asp, Glu, Asn, Aln, N_{ε}-Acetyllysin, N_{δ}-Acetylornithin, N_{γ}-Acetyldiaminobutyrat, N_{α}-Acetyldiaminobutyrat.

Die Kurzschreibweise der Aminosäuren erfolgte nach der allgemein üblichen Schreibweise. Die Di- oder Tripeptidreste sind in ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Bezüglich der Herstellung der Di- und Tripeptidreste wird ausdrücklich auf die EP 0 671 161 A1 der Firma Marbert verwiesen. Auch Beispiele für Di- und Tripeptidreste sind der Offenbarung der EP 0 671 161 A1 zu entnehmen.

Beispiele für C₁ - C₄-Alkylgruppen in den erfindungsgemäßen Verbindungen sind Methyl, Ethyl, n-Propyl, Isopropyl, n-Butyl, Isobutyl und tert.-Butyl. Bevorzugte Alkylgruppen sind Methyl und Ethyl, Methyl ist eine besonders bevorzugte Alkylgruppe. Bevorzugte C₂ - C₄-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxyalkylgruppe.

Die erfindungsgemäßen farbverändernden Mittel enthalten diese Wirkstoffe bevorzugt in Mengen von 0,001 bis 2, insbesondere von 0,01 bis 0,5 Gew.-%, jeweils bezogen auf die gesamte Anwendungszubereitung.

Im Rahmen einer neunten bevorzugten Ausführungsform enthalten die erfindungsgemäßen farbverändernden Mittel als Pflegestoff mindestens ein Mono- bzw. Oligosaccharid.

Es können sowohl Monosaccharide als auch Oligosaccharide, wie beispielweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Weiterhin können auch Derivate dieser Pentosen und Hexosen, wie die entsprechenden On- und

Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Weiterhin hat es sich als vorteilhaft erwiesen, wenn die erfindungsgemäßen Zubereitungen eine erhitzte, das heißt karamellisierte Zuckerart, vorzugsweise karamellisierten Rohrzucker enthalten.

Die Mono- bzw. Oligosaccharide sind in den erfindungsgemäßen Haarbehandlungsmitteln bevorzugt in einer Menge von 0,1 bis 8 Gew.-%, insbesondere 1 bis 5 Gew.-%, bezogen auf die gesamte Anwendungszubereitung, enthalten.

Im Rahmen einer zehnten Ausführungsform enthält die erfindungsgemäße Zubereitung (B) als Pflegestoff mindestens ein Silikonöl und/oder ein Silikongum.

Erfindungsgemäß geeignete Silikone oder Silikongums sind insbesondere Dialkyl- und Alkylarylsiloxane, wie beispielsweise Dimethylpolysiloxan und Methylphenyl-polysiloxan, sowie deren alkoxylierte, quaternierte oder auch anionische Derivate.

Gemäß einer bevorzugten Ausführungsform enthalten die erfindungsgemäßen Zubereitungen eine Kombination aus einem flüchtigen und einem nichtflüchtigen Silikon. Flüchtig im Sinne der Erfindung sind solche Silikone, die ein Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning^{®}1401, Dow Corning^{®}1403 und Dow Corning^{®}1501, jeweils Mischungen aus einem Cyclomethicone und einem Dimethiconol) erhältlich.

Gemäß einer besonders bevorzugten Ausführungsform wird als Komponente (A) ein Dialkylpolysiloxan oder eines seiner Derivate eingesetzt. Bevorzugt sind die Alkylgruppen Methyl, Ethyl, i-Propyl und n-Propyl. Dimethylpolysiloxan oder eines seiner Derivate wird besonders bevorzugt eingesetzt. Bevorzugte sind die Derivate des Dimethylplysiloxans, die aminofunktionell sind. Ein ganz besonders bevorzugtes Derivat ist unter der INCI-Bezeichnung Amodimethicone im Handel erhältlich.

Die erfindungsgemäßen Zubereitungen enthalten die Silikone bevorzugt in Mengen von 0,01 - 10 Gew.-%, insbesondere 0,1 - 5 Gew.-%, bezogen auf die gesamte Anwendungszubereitung.

Im Rahmen einer elften Ausführungsform enthalten die erfindungsgemäßen farbverändernden Mittel mindestens ein Lipid als Pflegestoff.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben.

Die erfindungsgemäßen Zubereitungen enthalten die Lipide bevorzugt in Mengen von 0,01 - 10 Gew.-%, insbesondere 0,1 - 5 Gew.-%, bezogen auf die gesamte Anwendungszubereitung.

Im Rahmen einer zwölften Ausführungsform enthalten die erfindungsgemäßen farbverändernden Mittel mindestens ein Ölkörper und/oder einen Wachs als Pflegestoff.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Din-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Diisotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin, Fettsäurepartialglyceride, das sind Monoglyceride, Diglyceride und deren technische Gemische. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.
   Die Einsatzmenge der natürlichen und synthetischen kosmetischen Ölkörper in den erfindungsgemäßen farbverändernden Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.
   Weiterhin hat es sich als vorteilhaft erwiesen, wenn die erfindungsgemäßen Mittel mindestens eine Esterverbindung enthalten, die als Alkoholkomponente einen gesättigten oder ungesättigten, verzweigten oder unverzweigten Wachsalkohol enthält.
   Dabei haben sich insbesonder die gesättigten Wachsalkohole als bevorzugt erwiesen. Weiterhin haben sich die unverzweigten Wachsalkohole als bevorzugt erwiesen. Besonders bevorzugt sind die gesättigten und unverzweigten Wachsalkohole. Ebenfalls bevorzugt sind Wachsalkohole mit einer endständigen Hydroxygruppe.
   Beispiele für erfindungsgemäß bevorzugte Wachsalkohole sind Tetracosan-1-ol, Hexacosan-1-ol, Triacontan-1-ol sowie Hentriacontan-1-ol. Triacontan-1-ol ist erfindungsgemäß ganz besonders bevorzugt.
   Als weiteren Bestandteil enthält die erfindungsgemäße Esterverbindung mindestens eine Fettsäurekomponente.
   Die Fettsäurekomponente weist vorzugsweise 10 bis 30 Kohlenstoffatome, insbesondere 16 bis 26 Kohlenstoffatome auf. Dabei sind sowohl gesättigte als auch ungesättigte sowie verzweigte und unverzweigte Fettsäuren prinzipiell geeignet. Als bevorzugte Fettsäuren kommen insbesondere die unverzweigten gesättigten Fettsäuren, wie beispielsweise Tetradecansäure, Pentadecansäure, Palmitinsäure, Margarinsäure, Stearinsäure, Nonadecansäure, Arachinsäure, Behensäure, Tetracosansäure, Cerotinsäure sowie Triacontansäure in Frage. Palmitinsäure, Stearinsäure, Behensäure und Triacontansäure sind erfindungsgemäß besonders bevorzugt.
   Besonders vorteilhaft im Sinne der vorliegenden Erfindung ist es ferner, wenn mindestens zwei verschiedene Esterverbindungen in den erfindungsgemäßen Mitteln enthalten sind, wobei die erste Esterverbindung eine Fettsäurekomponente mit 12 bis 18 Kohlenstoffatomen und die zweite Esterverbindung eine Fettsäurekomponente mit 22 bis 30 Kohlenstoffatomen aufweist.
   Ein Beispiel für eine derartige Kombination sind Esterverbindungen auf Basis von Palmitinsäure und
   Cerotinsäure.
   Ganz besonders bevorzugte Esterverbindungen sind die Ester von Triacontan-1-ol mit Palmitinsäure und
   Cerotinsäure. Derartige Esterverbindungen oder auch deren Mischungen können beispielsweise aus natürlichem Bienenwachs gewonnen werden.
   Es hat sich aber erfindungsgemäß auch als vorteilhaft erwiesen, wenn das komplexe Gemisch des natürlichen Bienenwachses, das eine erfindungsgemäße Esterverbindung umfasst, als solches in den erfindungsgemäßen Mitteln zum Einsatz kommt.
   Als natürliche oder synthetische Wachse können ferner erfindungsgemäß eingesetzt werden feste Paraffine oder Isoparaffine, Carnaubawachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.
   Die Wirkstoffkombination, die aus den erfindungsgemäßen Flavonoiden und Bienenwachs gebildet wird, ist erfindungsgemäß besonders bevorzugt, da durch ihren Einsatz der Haaroberfläche und das Haarinnere überraschenderweise vermehrt gepflegt werden kann.
   Im Rahmen einer dreizehnten Ausführungsform enthalten die erfindungsgemäßen Zubereitungenein Enzym als Pflegestoff. Erfindungsgemäß besonders bevorzugte Enzyme sind ausgewählt aus einer Gruppe, die gebildet wird aus Proteasen, Lipasen, Transglutaminase, Oxidasen und Peroxidasen.
   Obwohl jeder der in den verschiedenen Ausführungsformen genannten Pflegestoffe für sich alleine bereits ein zufriedenstellendes Resultat ergibt, sind im Rahmen der vorliegenden Erfindung auch alle Ausführungsformen umfasst, in denen die farbverändernden Mittel mehrere Pflegestoffe auch aus verschiedenen Gruppen enthalten.
   Besonders bevorzugte Pflegestoffe im Sinne der vorliegenden Erfindung sind insbesondere Vitamin C, Ubiquinone, Panthenol und seine Derivate, Ectoin, Kamillenextrakt, Bisabolol, Olivenöl, Royal Gelee, Mung Bean Extrakt und Moringa Olifeira-Extrakt.
   Im Rahmen einer vierzehnten bevorzugten Ausführungsform der vorliegenden Erfindung enthält die Zubereitung weiterhin mindestens ein Flavonoid der Formel (I) 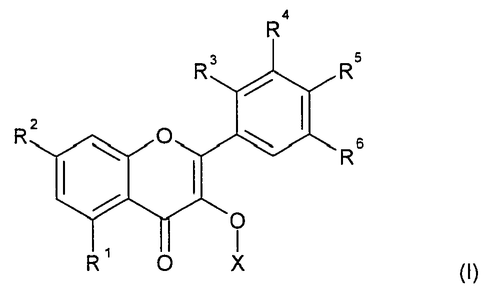 wobei R¹ bis R⁶ stehen unabhängig voneinander für ein Wasserstoffatom, eine Hydroxygruppe, ein Hydroxy-(C₁-bis C₄)-alkoxygruppe oder eine Sulfatgruppe und X steht für einen gegebenenfalls substituierten Glucosidrest, enthält.

Erfindungsgemäß bevorzugte Hydroxy-(C₁-bis C₄)-alkoxygruppen sind die Hydroxymethoxygruppe, die 2-Hydroxyethoxygruppe, die 3-Hydroxypropoxygruppe, die 2-Hydroxypropoxygruppe, die 4-

Hydroxybutoxygruppe, die 3-Hydroxybutoxygruppe sowie die 2-Hydroxybutoxygruppe. Die 2-Hydroxyethoxygruppe ist eine ganz besonders bevorzugte Hydroxy-(C₁-bis C₄)-alkoxygruppe.

Erfindungsgemäß haben sich Flavonoide der Formel (I) als bevorzugt erwiesen, bei denen mindestens zwei der Reste R¹ bis R⁶ für ein Wasserstoffatom stehen, besonders bevorzugt sind Flavonoide der Formel (I), bei denen zwei oder drei der Reste R¹ bis R⁶ für ein Wasserstoffatom stehen.

Weiterhin sind Flavonoide der Formel (I) bevorzugt, bei denen R¹ für eine Hydroxygruppe steht.

Ebenfalls bevorzugt sind Flavonoide, bei denen die Reste R¹ bis R⁶ maximal für 3 voneinander verschiedene Substituenten stehen, vorzugsweise lediglich für 2 voneinander verschiedene Substituenten.

Besonders bevorzugt sind Flavonoide der Formel (I), bei denen R¹, R² und R⁵ für eine Hydroxygruppe und R³, R⁴ und R⁶ für ein Wasserstoffatom stehen.

Weiterhin besonders bevorzugt sind Flavonoide der Formel (I), bei denen R¹ für eine Hydroxygruppe steht, R², R⁴ und R⁵ für eine Sulfatgruppe stehen und R³ und R⁶ für ein Wasserstoffatom stehen.

Ebenso besonders bevorzugt sind Flavonoide der Formel (I), bei denen R¹ für eine Hydroxygruppe steht, R², R⁴ und R⁵ für eine 2-Hydroxyethoxygruppe stehen und R³ und R⁶ für ein Wasserstoffatom stehen.

Ganz besonders bevorzugt sind Flavonoide der Formel (I), die ein durch die Gruppe X substituiertes Derivat von Flavonol, Galangin, Fisetin, Kaempferol, Quercetin, Morin, Robinetin, Gossypetin oder Myricetin sind.

Im Rahmen der erfindungsgemäßen Flavonoide der Formel (I) steht der Rest X für einen Glucosidrest. Dies ist erfindungsgemäß nicht auf Derivate der Glucose beschränkt, sondern steht ganz allgemein für αglucosidisch oder β-glucosidisch gebildete Acetale auch anderer Zuckerkomponenten.

Erfindungsgemäß bevorzugt haben sich Flavonoide der Formel (I) erwiesen, bei denen X für einen Rest der Formel (II) 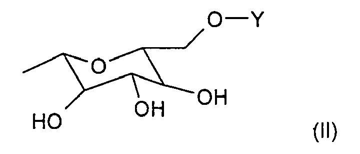 steht, wobei die Gruppe -O-Y für einen über eine Etherfunktion verknüpften weiteren Monosaccharidrest oder für einen über eine Esterfunktion verknüpften organischen aromatischen Säurerest steht.

Als erfindungsgemäße Monosaccharide kommen vorzugsweise sowohl Pentosen als auch Hexosen in Frage. Erfindungsgemäß bevorzugte Monosaccharidreste sind Ribose, Xylose, Arabinose, Glucose, Mannose, Galactose, Fructose, Sorbose, Fucose und Rhamnose. In einigen Fällen hat es sich als besonders bevorzugt erwiesen, wenn der Rest -O-Y für einen über eine Etherfunktion verknüpften 6-Desoxyzuckerrest, insbesondere einen Fucoserest oder einen Rhamnoserest, ganz besonders für einen Rhamnoserest, steht.

Im Rahmen einer anderen Ausführungsform der vorliegenden Erfindung steht die Gruppe -O-Y einen über eine Esterfunktion verknüpften organischen aromatischen Säurerest. Erfindungsgemäß bevorzugte gegebenenfalls substituierten Zimtsäurerest sind beispielsweise der o-Cumarsäurerest, der p-Cumarsäurerest und deren substituierten Derivate; der p-Cumarsäurerest ist ein ganz besonders bevorzugter Rest einer erfindungsgemäßen organischen aromatischen Säure.

Erfindungsgemäß ganz bsonders bevorzugte Flavonoide der Formel (I) sind
- Tilirosid ( [(2R,3R,4S,5R,6S)-6-[5,7-dihydroxy-2-(4-hydroxyphenyl)-4-oxo-chromen-3 -yl]oxy-3,4,5-trihydroxy-oxan-2-yl]methyl (E)-3-(4-hydroxyphenyl)prop-2-enoate; INCI-Bezeichnung: Tiliroside, CAS-Nr. 20316-62-5),
- Rutinsulfat (3,3',4', 4,7-Pentahydroxyflavon-3-rhamnoglucosid, INCI-Bezeichnung Disodium Rutinyl Disulfate),
- Troxerutin (2-[3,4-bis(2-hydroxyethoxy)phenyl]-5-hydroxy-7-(2-hydroxyethoxy)-3-[3, 4,5-trihydroxy-6-[(3,4,5-trihydroxy-6-methyl-oxan-2-yl)oxymethyl]oxan-2-yl]oxy-chromen-4-one; INCI-Bezeichnung: Troxerutin, CAS-Nr. 96304-71-1) und
- Isoquercetin (2-(3,4-dihydroxyphenyl)-5,7-dihydroxy-3[(2S,3R,4S,5R,6R)-3,4,5-trihydroxy-6-(hydroxymethyl)oxan-2-yl]oxy-chromen-4-one; CAS-Nr. 482-35-9).

Ein erfindungsgemäß ganz besonders bevorzugtes Flavonoid ist Tiliroside.

In der WO 02/69926 wird angegeben, dass Tilirosid beispielsweise aus den Pflanzen der Gattung Althaea, Aristolochia, Helianthemum, Lindera, Magnolia, Platanus, Potentilla, Quercus, Rosa, Sida, Sorbus und/oder Tilia gewonnen werden kann, wobei folgende Arten bevorzugt sind: Althaea officinalis, Althaea rosea, Aristolochia heterophylla, Helianthemum glomeratum, Lindera megaphyfla, Magnolia salicifolia, Platanus acerifolia, Platanus occidentalis, Potentilla anserina, Quercus pubescens, Quercus suber, Quercus laurifolia, Quercus ilex, Quercus imbricaria, Quercus virginiana, Rosa pomifera, Sida rhombifolia, Sida poeppigiana, Sida cordifolia, Sida glaziovii, Sorbus pendula, Tilia argenta und Tilia cordata.

In der WO-06/099930 wird weiterhin beschrieben, dass Tilirosid aus Pflanzen der Familie der Sterculiaceae gewonnen werden kann. Als bevorzugt werden dort die Waitheria Spezies, vorzugsweise Waitheria americana, Waitheria douradinha, Waitheria panicucata, Waitheria indica, Waitheria viscosissima, Waitheria antennalis, Waitheria ovata, Waitheria tornentosa, Waitheria madagascariensis, Waitheria glornerata, Waitheria bicolor, Waitheria fryxellii, Waitheria tundeltiana, Waitheria tridentata, Waitheria operculata, Waitheria bracteosa, Waitheria douradinha, Waitheria macropoda, Waitheria caroliniana, Waltheria arenicola, Waitheria melochia, Waitheria acurninata, Waitheria theobroma, Waitheria indivia oder Waitheria taiwana genannt.

Entsprechende Verfahren zur Gewinnung des Rohstoffs sind in den beiden genannten Schriften offenbart.

Tilirosid wird als kosmetischer Wirkstoff beispielsweise unter der Handelsbezeichnung Ronacare^{®} Tiliroside von der Fa. Merck kommerziell vertrieben. Es handelt sich dabei um eine Mischung von ca. 5Gew.-% reines Tilirosid mit ca. 95Gew,-% Sorbitol.

Auch wenn die Abmischung mit Sorbitol erfindungsgemäß bevorzugt ist, ist auch ein Einsatz von reinem Tilirosid oder in Kombination mit anderen pulverförmigen Stellmitteln erfindungsgemäß möglich.

Die Flavonoide der Formel (I) sind in den erfindungsgemäßen Zubereitungen vorzugsweise in einer Menge von 0,0001 bis 5Gew.-%, insbesondere von 0,002 bis 1Gew.-%, jeweils bezogen auf den reinen Wirkstoff und das gesamte anwendungsbereite Mittel, enthalten.

Besonders bevorzugt sind Mittel zur Färbung keratinischer Fasern, die neben dem erfindungsgemäßen Bräunungsprodukt, ein Flavonoid sowie Ectoin und/oder ein Ectoinderivat enthalten. Mittel, die neben dem Bräunungsprodukt das Flavonoid Tiliroside sowie Ectoin enthalten, sind ganz besonders bevorzugt

Die erfindungsgemäßen farbverändernden Mittel können neben den erfindungswesentlichen Komponenten weiterhin alle für solche Zubereitungen bekannten Wirk-, Zusatz- und Hilfsstoffe enthalten.

In vielen Fällen enthalten die farbverändernden Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Hinsichtlich der kationischen Tenside sei an dieser Stelle auf die obigen Ausführungen verwiesen.

Ferner können die erfindungsgemäßen farbverändernden Mittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
   enthalten.

Die farbverändernden Mittel enthalten die erfindungswesentlichen Komponenten erfindungsgemäß bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Weiterhin können die erfindungsgemäßen farbverändernden Mittel ein Reduktionsmittel enthalten. Beispiele für erfindungsgemäß bevorzugte Reduktionsmittel sind Natriumsulfit, Ascorbinsäure, Thioglykolsäure und deren Derivate, Natriumthionit, Alkalimetallcitratsalze und N-Acetyl-L-Cystein Ganz besonders bevorzugte Reduktionsmittel sind Alkalimetallcitratsalze, insbesondere Natriumcitrat, und N-Acetyl-L-Cystein. N-Acetyl-L-Cystein ist ein ganz besonders bevorzugtes Reduktionsmittel.

Weiterhin können die erfindungsgemäßen Mittel Alkalisierungsmittel, üblicherweise Alkali- oder Erdalkalihydroxide, Ammoniak oder organische Amine, enthalten. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und

Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Häufig werden in Färbemittel Perlglanzpigmente eingesetzt. Erfindungsgemäß bevorzugte Perlglanzpigmente sind natürliche Perlglanzpigmente wie z.B. Fischsilber (Guanin/Hypoxanthin-Mischkristalle aus Fischschuppen) oder Perlmutt (aus vermahlenen Muschelschalen), monokristalline Perlglanzpigmente wie z.B. Bismutoxychlorid, sowie Perlglanzpigmente auf Basis von Glimmer oder Glimmer/Metalloxid. Durch den Einsatz der Perlglanzpigmente werden Glanz und gegebenenfalls zusätzlich Farbeffekte in den erfindungsgemäßen Mitteln erzielt. Die Farbgebung durch die in den Mitteln verwendeten Perlglanzpigmente beeinflusst das Farbergebnis der Färbung der Keratinfasern jedoch nicht.

Perlglanzpigmente auf Glimmer-Basis und auf Glimmer/Metalloxid-Basis sind erfindungsgemäß ebenfalls bevorzugt. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet. Geeignete Metalloxide sind u.a. TiO₂, Cr₂O₃ und Fe₂O₃. Durch entsprechende Beschichtung werden Interferenzpigmente sowie Farbglanzpigmente als erfindungsgemäße Perlglanzpigmente erhalten. Diese Perlglanzpigmentarten weisen neben einem glitzernden optischen Effekt zusätzlich Farbeffekte auf. Desweiteren können die erfindungsgemäß verwendbaren Perlglanzpigmente weiterhin ein Farbpigment enthalten, welches sich nicht von einem Metalloxid ableitet.

Die Korngröße der bevorzugt verwendeten Perlglanzpigmente liegt bevorzugt zwischen 1.0 und 100 µm, besonders bevorzugt zwischen 5.0 und 60.0 µm.

Besonders bevorzugte Perlglanzpigmente sind Pigmente, die von der Firma Merck unter den Handelsnamen Colorona^{®} vermarktet werden, wobei die Pigmente Colorona^{®} red-brown (47-57 Gew.% Muscovit Mica (KH₂(AlSiO₄)₃), 43-50 Gew.% Fe₂O₃ (INCI: Iron Oxides Cl 77491), <3 Gew.% TiO₂ (INCI:

Titanium Dioxide Cl 77891), Colorona^{®} Blackstar Blue (39-47 Gew.% Muscovit Mica (KH₂(AlSiO₄)₃), 53-61 Gew.% Fe₃O₄ (INCI: Iron Oxides Cl 77499)), Colorona^{®} Siena Fine (35-45 Gew.% Muscovit Mica (KH₂(AlSiO₄)₃), 55-65 Gew.% Fe₂O₃ (INCI: Iron Oxides Cl 77491)), Colorona^{®} Aborigine Amber (50-62 Gew.% Muscovit Mica (KH₂(AlSiO₄)₃), 36-44 Gew.% Fe₃O₄ (INCI: Iron Oxides Cl 77499), 2-6 Gew.% TiO₂ (INCI: Titanium Dioxide Cl 77891)), Colorona^{®} Patagonian Purple (42-54 Gew.% Muscovit Mica (KH₂(AlSiO₄)₃), 26-32 Gew.% Fe₂O₃ (INCI: Iron Oxides Cl 77491), 18-22 Gew.% TiO₂ (INCI: Titanium Dioxide Cl 77891), 2-4 Gew.% Preussisch Blau (INCI: Ferric Ferrocyanide Cl 77510)), Colorona^{®} Chameleon (40-50 Gew.% Muscovit Mica (KH₂(AlSiO₄)₃), 50-60 Gew.% Fe₂O₃ (INCI: Iron Oxides Cl 77491)) und Silk^{®} Mica ( >98 Gew.% Muscovit Mica (KH₂(AlSiO₄)₃))_{.}

Erfolgt die Ausbildung der eigentlichen Färbung im Rahmen eines oxidativen Prozesses, so können übliche Oxidationsmittel wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Das eigentliche oxidative Färbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 5 bis 14, insbesondere von 7 bis 12, aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Erfindung ist ein Verfahren zur Färbung keratinischer Fasern, bei dem ein erfindungsgemäßes Mittel unmittelbar vor der Anwendung auf den Fasern mit einer Oxidationsmittelzubereitung vermischt wird, die resultierende Anwendungszubereitung auf die Fasern aufgetragen wird und nach einer Einwirkzeit wieder abgespült wird.

Es hat sich dabei als bevorzugt erweisen, wenn die Einwirkzeit von 1 bis 60 Minuten, vorzugsweise 30 bis 45 Minuten, beträgt.

Ein dritter Gegenstand der vorliegenden Erfindung ist die Verwendung eines aus Zucker durch Erhitzung gewonnenen Bräunungsproduktes als Anfärbefarbstoff für Haarbehandlungsmittel in Form von Emulsionene, Gelen und/oder alkoholischen Lösungen.

Im Rahmen dieses Gegenstandes hat es sich als vorteilhaft erwiesen, wenn das Haarbehandlungsmittel einen pH-Wert von 7 bis 14, vorzugsweise von 9 bis 12 aufweist. Weiterhin hat es sich als vorteilhaft erweisen, wenn das Haarbehandlungsmittel mindestens ein Farbstoffvorprodukt enthält.

Ein vierter Gegenstand der vorliegenden Erfindung ist die Verwendung eines aus Zucker durch Erhitzung gewonnenen Bräunungsproduktes zur Maskierung unangenehmer Gerüche von Haarbehandlungsmittein. Im Rahmen des dritten und vierten Gegenstandes der vorliegenden Erfindung hat es sich als vorteilhaft erwiesen, wenn es sich bei dem Haarbehandlungsmittel um ein Wellmittel, eine Fixiermittel, ein Blondiermittel oder ein oxidatives Färbemittel handelt.

Unter "Wellmittel" wird erfindungsgemäß eine wässrige Zubereitung einer keratinreduzierenden Substanz und unter "Fixiermittel" eine wässrige Zubereitung des Oxidationsmittels verstanden, die beide im Rahmen einer Dauerwellbehandlung zum Einsatz kommen.

Die erfindungsgemäßen Wellmittel enthalten zwingend mindestens ein keratinreduzierende Substanz, vorzugsweise Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Salze der schwefligen Säure. Bevorzugt geeignet sind die Alkali- oder Ammoniumsalze der Thioglykolsäure und/oder der Thiomilchsäure sowie die freien Säuren. Diese werden in den Wellmitteln bevorzugt in Konzentrationen von 0,5 bis 1,0 mol/kg bei einem pH-Wert von 5 bis 12, insbesondere von 7 bis 9,5, eingesetzt. Zur Einstellung dieses pH-Wertes enthalten die erfindungsgemäßen Wellmittel üblicherweise Alkalisierungsmittel wie Ammoniak, Alkali- und Ammonium-carbonate und - hydrogencarbonate oder organische Amine wie Monoethanolamin.

Weiterhin können die erfindungsgemäßen Wellmittel wellkraftverstärkende Komponenten enthalten, wie beispielsweise
- heterocyclische Verbindungen wie Imidazol, Pyrrolidin, Piperidin, Dioxolan, Dioxan, Morpholin und Piperazin sowie Derivate dieser Verbindungen wie beispielsweise die C₁₋₄-Alkyl-Derivate, C₁₋₄-Hydroxyalkyl-Derivate und C₁₋₄-Aminoalkyl-Derivate. Bevorzugte Substituenten, die sowohl an Kohlenstoffatomen als auch an Stickstoffatomen der heterocyclischen Ringsysteme positioniert sein können, sind Methyl-, Ethyl-, β-Hydroxyethyl- und β-Aminoethyl-Gruppen. Erfindungsgemäß bevorzugte Derivate heterocyclischer Verbindungen sind beispielsweise 1-Methylimidazol, 2-Methylimidazol, 4(5)-Methylimidazol, 1,2-Dimethylimidazol, 2-Ethylimidazol, 2-Isopropylimidazol, N-Methylpyrrolidon, 1-Methylpiperidin, 4-Methylpiperidin, 2-Ethylpiperidin, 4-Methylmorpholin, 4-(2-Hydroxyethyl)morpholin, 1-Ethylpiperazin, 1-(2-Hydroxyethyl)piperazin, 1-(2-Aminoethyl)piperazin. Weiterhin erfindungsgemäß bevorzugte Imidazolderivate sind Biotin, Hydantoin und Benzimidazol. Ganz besonders bevorzugt ist das Imidazol.
- Aminosäuren wie insbesondere Arginin, Citrullin, Histidin, Ornithin und Lysin. Die Aminosäuren können sowohl als freie Aminosäure als auch als Salze, z. B. als Hydrochloride, eingesetzt werden. Weiterhin haben sich auch Oligopeptide aus durchschnittlich 2-3 Aminosäuren, die einen hohen Anteil (> 50 %, insbesondere > 70 %) an den genannten Aminosäuren haben, als erfindungsgemäß einsetzbar erwiesen. Erfindungsgemäß besonders bevorzugt sind Arginin sowie dessen Salze und argininreiche Oligopeptide.
- Diole wie beispielsweise 2-Ethyl-1,3-hexandiol, 1,3-Butandiol, 1,4-Butandiol, 1,2-Propandiol, 1,3-Propandiol, Neopentylglykol und Ethylenglykol. 1,3-Diole, insbesondere 2-Ethyl-1,3-hexandiol und 1,3-Butandiol, haben sich als besonders gut geeignet erwiesen.
   Bezüglich näherer Informationen zu solchen wellkraftverstärkenden Komponenten wird auf die Druckschriften DE-OS 44 36 065 und EP-B1-363 057 verwiesen, auf deren Inhalt ausdrücklich Bezug genommen wird.

Die wellkraftverstärkenden Verbindungen können in den erfindungsgemäßen Wellmitteln in Mengen von 0,5 bis 5 Gew.-%, bezogen auf das gesamte Wellmittel, enthalten sein. Mengen von 1 bis 4 Gew.-%, im Falle der Diole von 0,5 bis 3 Gew.-%, haben sich als ausreichend erwiesen, weshalb diese Mengen besonders bevorzugt sind.

Zwingender Bestandteil der erfindungsgemäßen Fixiermittel sind Oxidationsmittel, z. B. Natriumbromat, Kaliumbromat, Wasserstoffperoxid, und die zur Stabilisierung wässriger Wasserstoffperoxidzubereitungen üblichen Stabilisatoren. Der pH-Wert solcher wässriger H₂O₂-Zubereitungen, die üblicherweise etwa 0,5 bis 15 Gew.-%, gebrauchsfertig in der Regel etwa 0,5 bis 3 Gew.-%, H₂O₂ enthalten, liegt bevorzugt bei 2 bis 6, insbesondere 2 bis 4; er wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (z. B. Peroxidasen), die keine oder nur geringe Mengen an Oxidationsmitteln, insbesondere H₂O₂, enthalten.

Die erfindungsgemäßen Wellmittel beziehungsweise Fixiermittel sind üblicherweise einphasig formuliert, eingeschlossen von diesem Begriff sind dabei Systeme, die eine kontinuierliche Phase aufweisen, wie beispielsweise reine ÖI-in-Wasser- oder Wasser-in-ÖI-Emulsionen.

Es hat sich gezeigt, das erfindungsgemäß auch Zwei- und Mehrphasensysteme bevorzugt sind. Dies sind Systeme, bei denen mindestens zwei separate, kontinuierliche Phasen vorliegen. Beispiele für solche Systeme sind Zubereitungen, die folgende Phasen aufweisen:
- eine wässrige Phase und eine nichtwässrige Phase, die separat voneinander vorliegen
- eine wässrige Phase und zwei nichtwässrige, miteinander nicht mischbare Phasen, die jeweils separat vorliegen
- eine Öl-in-Wasser-Emulsion und eine davon separiert vorliegende nichtwässrige Phase
- eine Wasser-in-ÖI-Emulsion und eine davon separiert vorliegende wässrige Phase.

Unter "Blondiermittel" werden üblicherweise feste oder pastenförmige Zubereitungen mit festen Peroxoverbindungen verstanden, die unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die festen Peroxoverbindungen stellen in der Regel keine Anlagerungsprodukte von Wasserstoffperoxid an andere Komponenten dar. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat und Peroxide wie Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Peroxoverbindungen sind in den erfindungsgemäßen Mitteln bevorzugt in Mengen von 5-30 Gew.-%, insbesondere in Mengen von 8-20 Gew.-%, enthalten.

Als weitere wichtige Komponente enthalten Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxide, -carbonate, -hydrogencarbonate, -hydroxycarbonate, -silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen beispielsweise aus einem Metasilikat und einem Hydroxycarbonat bevorzugt sein.

Blondiermittel enthalten Alkalisierungsmittel bevorzugt in Mengen von 5-25 Gew.-%, insbesondere 10-20 Gew.-%.

Blondiermittel enthalten üblicherweise als weitere wichtige Komponente Wasserstoffperoxid oder eine feste Anlagerungsverbindung von Wasserstoffperoxid an anorganische oder organische Verbindungen, wie beispielsweise Natriumperborat, Natriumpercarbonat, Natriumpercarbamid, Polyvinylpyrrolidon, Harnstoffperoxid und Melaminperoxid.

Die Konzentration dieser Wasserstoffperoxid-Lösung wird einerseits von den gesetzlichen Vorgaben und andererseits von dem gewünschten Effekt bestimmt; in der Regel werden 6- bis 12-prozentige Lösungen in Wasser verwendet. Die Mengenverhältnisse von der Komponente, die die Peroxoverbindung enthält zur Wasserstoffperoxid-Lösung liegt dabei üblicherweise im Bereich 1:1 1 bis 1:2, wobei ein Überschuss an Wasserstoffperoxid-Lösung insbesondere dann gewählt wird, wenn keine zu ausgeprägte Blondierwirkung erwünscht ist.

Bezüglich bevorzugter Einsatzkonzentrationen, bevorzugter weiterer Inhaltsstoffe der jeweiligen verwendeten Mittel und weitere bevorzugter Ausführungsformen des zweiten bis vierten Gegenstandes gilt mutatis mutandis das zu den erfindungsgemäßen Mitteln Gesagte.

Die nachfolgenden Beispiele sollen den Gegenstand der vorliegenden Anmeldung verdeutlichen ohne ihn in irgendeiner Weise zu beschränken.

### Beispiele

Die Mengenangaben verstehen sich, soweit nichts anderes vermerkt ist, jeweils in Gewichtsprozent.

### 1 Färbemittel

### 1.1 Herstellung der Farbrezepturen

| Rohstoff | Farbrezeptur A | Farbrezeptur B | FarbrezepturC | Farbrezeptur D |
|---|---|---|---|---|
| Xanthan^{®} FN | 0,1 | 0,1 | 0,1 | 0,1 |
| Ectoin | 0,1 | 0,5 | 1,2 | 2,0 |
| Ronacare^{®} Tiliroside | 0,01 | 2,0 | 0,5 | 0,05 |
| Cetiol^{®} V | 2,3 | 2,3 | 2,3 | 2,3 |
| Lanette^{®} N | 14,0 | 14,0 | 14,0 | 14,0 |
| Cetearyl Alcohol | 3,9 | 3,9 | 3,9 | 3,9 |
| Cutina GMS SE | 6,0 | 6,0 | 6,0 | 6,0 |
| Kokosamidopropylbetain 40 % | 2,0 | 2,0 | 2,0 | 2,0 |
| Monoethanolamin | 0,2 | 0,4 | 0,3 | 0,3 |
| Karamelzuckersirup 75 % | 0,1 | 0,1 | 0,1 | 0,1 |
| Ascorbinsäure | 0,1 | 0,1 | 0,1 | 0,1 |
| Natriumsulfit | -- | 0,3 | -- | 0,2 |
| Monoethanolamin | 3,0 | 3,0 | 3,0 | 3,0 |
| p-Toluylendiaminsulfat | 0,7 | 1,5 | 1,0 | 1,0 |
| m-Aminophenol | 0,1 | 0,2 | 0,1 | 0,04 |
| 2-Amino-4-Hydroxyethylaminoanisolsulfat | 0,04 | 0,02 | -- | -- |
| Resorcin | 0,3 | 0,6 | 0,3 | 0,1 |
| 2-Methylresorcin | -- | -- | 0,1 | 0,3 |
| 2-Amino-3-hydroxypyridin | -- | -- | -- | 0,1 |
| Wasser | ad 100 | ad 100 | ad 100 | ad 100 |

Die Färbecremes wiesen eine angenehme beige-Färbung auf.

### 1.2 Herstellung der Oxidationsmittelzubereitung

| Rohstoff | Menge |
|---|---|
| Natriumbenzoat | 0,04 |
| Dinatriumpyrophosphat | 0,1 |
| Turpinal^{®} SL | 0,3 |
| Kaliumhydroxid 50 % | 0,1 |
| Isopropylmyristat | 0,8 |
| Cetearyl Alcohol | 4,0 |
| Eumulgin^{®} B 2 | 1,0 |
| Paraffinum Liquidum | -- |
| Bienenwachs | 0,5 |
| Meadowfoam Seed Oil | -- |
| Wasserstoffperoxid (50%ig) | 5,2 |
| Wasser | ad 100 |

### 1.3 Ausfärbungen

50g der Farbrezepturen A bis D wurden jeweils mit 100g der Oxidationsmittelzubereitung gemischt. Die resultierenden Anwendungszubereitungen wurden jeweils auf eine Strähne Büffelbauchhaar aufgetragen und dort für 30 Minuten bei Raumtemperatur belassen.

Anschließend wurden die Haare gründlich mit einem handelsüblichen Shampoo gespült.

### 2 Weitere Haarbehandlungsmittel

### 2.1 Haarconditioner

| Rohstoff | Menge |
|---|---|
| Isopropylmyristat | 1,0 |
| Cutina^{®} GMS-V | 0,8 |
| Dehyquart^{®} F 75 | 1,0 |
| Varisoft^{®} W 575 PG | 2,5 |
| Cetearyl Alcohol | 5,3 |
| Eumulgin^{®} B 2 | 0,6 |
| Gluadin^{®} WQ | 0,2 |
| Methylparaben | 0,2 |
| Stearamidopropyldimethylamin | 0,8 |
| Citronensäure-Monohydrat | 0,6 |
| Cosmedia^{®} CTH (E) | 0,6 |
| Colorona^{®} Precious Gold | 0,3 |
| Karamelzuckersirup 75 % | 0,3 |
| Amber Extract M.S. (4667) | 0,3 |
| ProSina^{®} | 0,1 |
| D-Panthenol 75 % | 0,2 |
| Dow Corning^{®} 1403 Fluid | 2,0 |
| Phenoxyethanol | 0,4 |
| Parfum | 0,5 |
| Wasser | ad 100 |

### 2.2 Haarkur

| Rohstoff | Menge |
|---|---|
| Polymer^{®} JR 400 | 0,02 |
| Synthalen^{®} CR | 2,2 |
| Dow Corning^{®} 949 | 1,0 |
| Gafquat^{®} 755 | 1,5 |
| Gluadin^{®} WQ | 0,2 |
| ProSina^{®} | 0,2 |
| D-Panthenol 75 % | 0,2 |
| Amber Extract M.S. (4667) | 0,3 |
| Timiron^{®} Super Silver | 0,1 |
| Colorona^{®} Precious Gold | 0,1 |
| Karamelzuckersirup 75 % | 0,4 |
| Milchsaeure 80% DAB | 0,05 |
| Trisiloxane Fluid 1 cs | 2,5 |
| Dow Corning^{®} 200 Fluid | 0,7 |
| Cosmedia^{®} CTH (E) | 0,3 |
| Parfum | 0,6 |
| Natronlauge 50% Standard | 0,02 |
| Ethanol 96 % DEP vergällt | 15,0 |
| Wasser | ad 100 |

### 2.3 Haartreatment

| Rohstoff | Menge |
|---|---|
| Cetearyl Alcohol | 5,8 |
| Varisoft^{®} W 575 PG | 1,0 |
| Cutina^{®} AGS | 1,0 |
| Dehyquart^{®} F 75 | 1,0 |
| Colorona^{®} Precious Gold | 0,4 |
| Methylparaben | 0,3 |
| Milchsaeure 80% DAB | 0,05 |
| Cosmedia^{®} CTH (E) | 0,4 |
| Quartamin^{®} BTC-131 | 1,5 |
| Gluadin^{®} WQ | 0,2 |
| Karamelzuckersirup 75 % | 0,2 |
| DC^{®} 200 FLUID 60000 CST. | 1,0 |
| Phenoxyethanol, rein | 0,4 |
| D-Panthenol 75 % | 0,2 |
| ProSina^{®} | 0,2 |
| Amber Extract M.S. (4667) | 0,3 |
| Parfum | 0,5 |
| Wasser | ad 100 |

### 3 Eingesetzte Rohstoffe

- Amber Extract M.S. (4667): Bernsteinextrakt, INCI-Bezeichnung: Propylene Glycol, Amber Extract, Alcohol Denat. (ProVital)
- Cetiol^{®} V: Ölsäuredecylester (INCI-Bezeichnung: Decyl Oleate) (Cognis)
- Colorona^{®} Precious Gold: Pigment aus 20-51% Mica, 15-25%SiO₂, 19-29% TiO₂, 15-25% Fe₂O₃; INCI-Bezeichnung: Mica, Cl 77891 (Titanium dioxide), Silica, Cl 77491(Iron oxides), Tin oxide; (Merck)
- Cosmedia^{®} CTH (E): ca. 50% Aktivsubstanzgehalt, INCI-Bezeichnung: Polyquaternium-37, Propylene Glycol Dicaprylate/Dicaprate,PPG-1 Trideceth-6 (Cognis)
- Cutina^{®} AGS: Ethylenglykoldistearat (INCI-Bezeichnung: Glycol Distearate) (Cognis)
- Cutina^{®} GMS-SE: INCI-Bezeichnung: Glyceryl Stearate SE (Cognis)
- Cutina^{®} GMS-V: Glycerinmono/dipalmitat/stearat (INCI-Bezeichnung: Glyceryl Stearate) (Cognis)
- Dehyquart^{®} F75: Fettalkohole-Methyltriethanolammoniummethylsulfat-dialkylester-Gemisch (INCI-Bezeichnung: Distearoylethyl Hydroxyethylmonium Methosulfate, Cetearyl Alcohol) (Henkel)
- Dow Corning 1403^{®} Fluid: Silikonmischung (100% Silikonanteil, davon ca. 13% Dimethiconol; INCI-Bezeichnung: Dimethicone, Dimethiconol) (Dow Corning)
- Dow Corning^{®} 200 Fluid: Polydimethylsiloxan (INCI-Bezeichnung: Dimethicone) (Dow Corning)
- Dow Corning^{®} 949: ca. 35% Festkörper; INCI-Bezeichnung: Amodimethicone, Trideceth-12, Cetrimonium Chloride (Dow Corning)
- Eumulgin^{®} B2: Cetylstearylalkohol mit ca. 20 EO-Einheiten (INCI-Bezeichnung: Ceteareth-20) (Cognis)
- Gafquat^{®} 755: Dimethylaminoethylmethacrylat-Vinylpyrrolidon-Copolymer, quaterniert mit Diethylsulfat (ca. 19% Festkörper in Wasser; INCI-Bezeichnung: Polyquaternium-11) (ISP)
- Gluadin^{®} WQ: Weizenproteinhydrolysat (ca. 31-35% Festkörper; INCI-Bezeichnung: Aqua (Water), Laurdimonium Hydroxypropyl Hydrolyzed Wheat Protein, Ethylparaben, Methylparaben) (Cognis)
- Lanette^{®} N: Gemisch aus 90 Gewichtsteilen Cetylstearylalkohol und 10 Gewichtsteilen Natriumcetylstearylsulfatl (INCI-Bezeichnung: Cetearyl Alcohol, Sodium Cetearyl Sulfate) (Cognis)
- Polymer JR^{®} 400: quaternierte Hydroxyethylcellulose (INCI-Bezeichnung: Polyquaternium-10) (Amerchol)
- ProSina^{®}: Keratinhydrolysat, INCI-Bezeichnung: Aqua (Water), Hydrolyzed Keratin (Croda)
- Quartamin^{®} BTC-131: ca. 50% QAV, INCI-Bezeichnung: Behenoyl PG-Trimonium Chloride (Kao)
- Ronacare^{®} Tiliroside: Mischung von Tiliroside und Sorbitol (ca. 4-5Gew% Tiliroside; INCI-Bezeichnung: Sorbitol, Tiliroside) (Merck)
- Synthalen^{®} CR: 2-(Trimethylammonio)ethylmethacrylatchloridhomopolymer (INCI-Bezeichnung: Polyquaternium-37) (3V Sigma)
- Timiron^{®} Super Silver: Perglanzpigment (INCI-Bezeichnung: Mica, Cl 77891 (Titanium Dioxide)) (Merck)
- Turpinal^{®} SL: 1-Hydroxyethan-1,1-diphosphonsäure (ca. 58 - 61% Aktivsubstanzgehalt; INCI-Bezeichnung: Etidronic Acid, Aqua (Water)) (Solutia)
- Varisoft^{®} W 575 PG: INCI-Bezeichnung: Quaternium-87 (Goldschmidt)
- Xanthan^{®} FN: INCI-Bezeichnung: Xanthan Gum (Jungbunzlauer)

## Patentansprüche

1. Mittel zum Färben keratinischer Fasern, **dadurch gekennzeichnet, dass** es mindestens ein Farbstoffvorprodukt sowie ein Bräunungsprodukt, das durch Erhitzung aus Zucker gewonnen wird, enthält und einen pH-Wert von 7 bis 14, vorzugsweise von 9 bis 12 aufweist.

2. Mittel nach Anspruch 1, **dadurch gekennzeichnet, dass** das Bräunungsprodukt aus Polysacchariden durch Zusatz von Bräunungsbeschleunigern gewonnen wird.

3. Mittel nach Anspruch 2, **dadurch gekennzeichnet, dass** als Bräunungsbeschleuniger Natriumcarbonat, Kaliumcarbonat, Natriumhydroxid, Kaliumhydroxid, Essigsäure, Citronensäure, Schwefelsäure, Schwefeldioxid, Ammoniak, Ammoniumcarbonat, Ammoniumsulfit, Natriumsulfit und/oder Kaliumsulfit verwendet wird.

4. Mittel nach Anspruch 3, **dadurch gekennzeichnet, dass** das Bräunungsprodukt ausgewählt ist aus E 150a, E 150b, E 150c und/oder E 150d.

5. Mittel nach Anspruch 1, **dadurch gekennzeichnet, dass** das Bräunungsprodukt ausschließlich durch Erhitzen eines oder mehrerer Zuckerarten ohne Zugabe eines Bräunungsbeschleunigers gewonnen wird.

6. Mittel nach einem der Ansprüche 1 bis 6, **dadurch gekennzeichnet, dass** es als Farbstoffvorprodukt mindestens ein Oxidationsfarbstoffvorprodukt vom Entwickler- und/oder Kupplertyp enthält.

7. Mittel nach einem der Ansprüche 1 bis 7, **dadurch gekennzeichnet, dass** es als Farbstoffvorprodukt eine Vorstufe eines naturanalogen Farbstoffs enthält.

8. Mittel nach einem der Ansprüche 1 bis 8, **dadurch gekennzeichnet, dass** es ein Alkalisierungsmittel ausgewählt aus Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol, Triethanolamin, Arginin, Alkali- und Erdalkalimetallhydroxiden enthält.

9. Verwendung eines aus Zucker durch Erhitzung gewonnenen Bräunungsproduktes als Anfärbefarbstoff für Haarbehandlungsmittel in Form von Emulsionen, Gelen und/oder alkoholischen Lösungen.

10. Verwendung nach Anspruch 9, **dadurch gekennzeichnet, dass** das Haarbehandlungsmittel einen pH-Wert von 7 bis 14, vorzugsweise von 9 bis 12 aufweist.

11. Verwendung nach einem der Ansprüche 9 oder 10, **dadurch gekennzeichnet, dass** das Haarbehandlungsmittel mindestens ein oxidatives Farbstoffvorprodukt enthält.

12. Verwendung eines aus Zucker durch Erhitzung gewonnenen Bräunungsproduktes zur Maskierung unangenehmer Gerüche von Haarbehandlungsmitteln.

13. Verwendung nach Anspruch 12 zur Maskierung von unangenehmen Gerüchen von Wellmitteln, Fixiermitteln, Blondiermitteln oder oxidativen Färbemitteln.

14. Verfahren zur Färbung keratinischer Fasern, **dadurch gekennzeichnet, dass** ein Mittel nach einem der Ansprüche 1 bis 8 unmittelbar vor der Anwendung auf den Fasern mit einer Oxidationsmittelzubereitung vermischt wird, die resultierende Anwendungsmischung auf die Fasern aufgetragen wird und nach einer Einwirkzeit wieder abgespült wird.
